# EUROPEAN PATENT SPECIFICATION

(11) **EP 2 214 638 B1**
(45) Date of publication and mention of the grant of the patent: **18.01.2012**
(21) Application number: 08845987.0
(22) Date of filing: 21.10.2008
(51) Int. Cl.: A61K 9/00

(54) **COMPOSITION COMPRISING POLYUNSATURATED FATTY ACIDS AND ACTIVATED CHARCOAL**
ZUSAMMENSETZUNG MIT MEHRFACH UNGESÄTTIGTEN FETTSÄUREN UND AKTIVIERTER HOLZKOHLE
COMPOSITION COMPRENANT DES ACIDES GRAS POLYINSATURÉS ET DU CHARBON ACTIF

(30) Priority: 30.10.2007 EP 07291306
(43) Date of publication of application: 11.08.2010
(73) Proprietor: Bayer Consumer Care AG, 4052 Basel (CH)
(72) Inventor: KABARADJIAN, Catherine, F-74100 Vétraz-Monthoux (FR)
(74) Representative: Albers, Markus
(86) International application number: PCT/EP2008/008882
(87) International publication number: WO 2009/056247

(56) References cited:
- WO-A-2007/015102
- DE-U1- 20 105 126
- KOBAYASHI KOJI ET AL: "The effect of n - 3 PUFA/gamma-cyclodextrin complex on serum lipids in healthy volunteers--a randomized, placebo-controlled, double-blind trial." ASIA PACIFIC JOURNAL OF CLINICAL NUTRITION 2007, vol. 16, no. 3, 2007, pages 429-434, XP002515873 ISSN: 0964-7058
- BENATTI PAOLA ET AL: "Polyunsaturated fatty acids: Biochemical, nutritional and epigenetic properties" JOURNAL OF THE AMERICAN COLLEGE OF NUTRITION, vol. 23, no. 4, August 2004 (2004-08), pages 281-302, XP002513251 ISSN: 0731-5724

## Description

The present invention relates to an oral composition without bad odour, smell or taste comprising polyunsaturated fatty acids (PUFA) and activated charcoal, its process for preparation and its use as nutritional supplement or as dietary supplement for balancing the blood lipid level, preventing or reducing the risk of the development of atherosclerotic changes, disorders or diseases.

Polyunsaturated fatty acids (PUFA) are the active ingredients e.g. in fish oil and are responsible for significant low blood lipid levels and low incidence of hypertension which was shown in a epidemiological study with Inuits (M.H. Davidson, P. R. Liebson, Cardiovascular Reviews&Reports 1986, 7, 461-472). In particular the blood concentration of the low density lipoprotein cholesterol (LDL) is lowered and that of the high density lipoprotein cholesterol (HDL) is increased. Coronary heart disease (CHD) is the major cause of death in the western countries and high plasma cholesterol levels, more specifically the LDL/HDL ratio, is highly correlated with the risk of CHD (Willett and Sacks, N. Eng. J. Med. 1991, 121, 324).

The PUFA found in fish oil have carbon chains of 18, 20 or 22 atoms and can be classified in n-3 omega and n-6 omega fatty acids which are essential for the human body. In particular the omega-3 fatty acids eicosapentenoic acid (EPA) and docosahexenoic acid (DHA) are only found in fish and other marine life. Fish oil is therefore the most important food source for omega-3 fatty acids.

Standard nutritional supplement of PUFA is achieved by a daily administration of 500-1000 mg liquid fish oil. The fish oil is normally administered in capsules in order to prevent the fishy smell and odour. Nevertheless some people experience gastrointestinal upset of the fishy smell even hours after the fish oil is taken. The reason is that after decomposition of the galantine wall in the gastro-intestinal tract, the voluminous dosage of the fish oil is released as a macroscopic drop which interfere with the resorption of the fish oil. The problems of bad smell, odour and resorption can be solved in part by microdispersed fish oil preparations as pulverulent or aqueous matrix as described in EP 0 276 772. However, even the microdispersed fish oil granule or powder has a remaining fishy smell when pressed in simple tablets, so that flavours or antioxidants are needed to be added to the formulations.

The flavours are used to domineer over the fishy smell of the fish oil as described e.g. in JP 08092587 or JP 08228678. Taste masking is also achieved by adding milk products to the fish oil formulation as described e.g. in US 2003/0198728 or JP 2002-204656 or by coating or encapsulating the PUFA as described e.g. in WO 2004/016720 and WO 2005/029978.

The reason for the fishy smell of the fish oil is based on the oxidation of the unsaturated part of the PUFA. In order to prevent oxidation and to stabilize the PUFA antioxidants e.g. tocopherol can be added to the formulation as described e.g. in DE 20105126 or EP 1 155 620.

The object of the present invention is to provide an oral composition containing PUFA, having no bad odour or smell and avoiding a complex and expensive taste masking technology.

Surprisingly it is found that a bad or fishy smell of the composition according to the present invention can be avoided.

Subject of the present invention is an oral composition comprising fish oil, perilla oil or polyunsaturated fatty acids (PUFA) and activated charcoal. The oral composition according to the invention has not a fishy or bad smell, odour or taste.

According to the present invention polyunsaturated fatty acids (PUFA) include, but are not limited to, those comprised in fish oil or perilla oil, or omega-3 fatty acids, omega-6-fatty acids, arachidonic acid, linoleic acid, alpha-linoleic acid, dihomogammalinoleic acid, eicosapentenoic acid (EPA), docosahexenoic acid (DHA) and mixtures thereof. Preference is given to fish oil comprising PUFA, omega-3 fatty acids, eicosapentenoic acid (EPA), docosahexenoic acid (DHA) and mixtures thereof. Most preferably fish oil containing eicosapentenoic acid (EPA) and docosahexenoic acid (DHA) is used.

In a preferred embodiment the composition according to the invention comprises fish oil, perilla oil or PUFA, in particular fish oil powder or granulate, in an amount of 5 % to 70%, more preferable 40 % to 60 % by weight of the composition. The total amount of the fish oil, perilla oil or PUFA, in particular fish oil powder or granulate, used in the composition is 100 mg - 1000 mg, preferably 400 mg - 900 mg.

In a most preferred embodiment the composition according to the invention comprises eicosapentenoic acid (EPA) in an amount of 0.5 % to 10 %, preferably 1.5 % to 4 % by weight of the composition or in an amount of 1 % to 20 %, preferably 3 % to 8 % by weight of the fish oil powder or granulate contained in the composition. The total amount of EPA in the composition is 10 mg - 100 mg, preferably 15 mg - 50 mg.

In a most preferred embodiment the composition according to the invention comprises docosahexenoic acid (DHA) in an amount of 0.5 % to 10 %, preferably 1 % to 4 % by weight of the composition or in an amount of 1 % to 20 %, preferably 2 % to 6 % by weight of the fish oil powder or granulate contained in the composition. The total amount of DHA in the composition is 10 mg - 50 mg, preferably 15 mg - 30 mg.

The PUFA, in particular the fish oil containing eicosapentenoic acid (EPA) and docosahexenoic acid (DHA), is used preferably in a microdispersed form as a granulate or powder, in a pulverulent or aqueous matrix as described in EP 0 276 772. Preference is given to a fish oil granulate or powder.

In a preferred embodiment the pulverulant matrix of the fish oil powder or granulate comprises at least a homogenous protective colloid, a surfactant, optionally a diluent, stabilizer and further pharmaceutical excipients. In the case of the aqueous matrix the mentioned ingredients are used as an aqueous solution.

The protective colloids of the pulverulant matrix include but are not limited to polypeptides such as gelatine, casein, caseinate, polysaccharides such as starch, dextrin, pectin, Arabic gum, milk, milk powder, polyvinyl alcohols, polyvinylpyrrolidone, vinylpyrrolidone-vinylacetate-copolymers, acrylic acid- and methacrylic acid-copolymers with acrylic acid- or methacrylic acid esters, methyl cellulose, carboxymethyl cellulose, hydroxypropyl cellulose, alginates or mixtures thereof.

Diluents of the pulverulant matrix include but are not limited to sugar or sugar alcohols e.g. saccharose, lactose, invert sugar, sorbit, mannit or glycerine.

Stabilizers of the pulverulant matrix include but are not limited to tocopherol, t-butyl hydroxytoluol, t-butyl hydroxyanisol and ethoxyquine.

Surfactants of the pulverulant matrix include but are not limited to esters of long chain fatty acids and ascorbic acid, esters of mono- and diglycerin and fatty acids and oxethylated derivatives thereof, esters of mono fatty acid glycerides with acetic acid, citric acid, lactic acid, diacetyltartrate, salts of 2-(2'-stearoyllactyl) lactic acid, polyglycerine fatty acid esters, sorbitan fatty acid esters, porpylenglycol-fatty acid esters, ascorbylpalmitate and lecithin.

The pulverulant matrix comprises fish oil in the amount of 5 % to 70 %, preferably 50 % to 60 %, one or more surfactants in an amount of 1 % to 30 %, preferably 5 % to 15 %, a protective colloid in an amount of 5 % to 50 %, preferably 10 % to 40 %, a diluent in an amount of 0 % to 70 %, preferably 3 % to 35 %, by weight of the dry mass of the fish oil powder or granulate.

In a preferred embodiment the pulverulant matrix comprises the fish oil in small particles having an average particle size of less then 10 µm, more preferably less then 1 µm, most preferably less then 0.5 µm.

The fish oil powder or granulate can be prepared as described in EP 0 276 772.

The composition according to the invention comprises activated charcoal in an amount of 0.1 % up to 10 %, preferably 1 % up to 5 %, by weight of the composition. The total amount used in the composition is 5 mg up to 200 mg, preferably 10 mg up to 100 mg.

The particles of the activated charcoal according to the present invention have preferably a portion of at least 90 % of particles having a particle size of less then 100 µm.

The function of the activated charcoal according to the invention is to adsorb the bad or fishy smell, odor or taste.

The composition according to the invention can comprise further active ingredients such as vitamins and minerals. Vitamins include, but are not limited to, vitamin A, beta carotene, vitamin C (ascorbic acid), vitamin D3 (cholecalcipherol), vitamin E (tocopherol acetate), vitamin B1 (thiamine), vitamin B2 (riboflavin), nicotinamide, vitamin B5 (panthothenic acid), vitamin B6 (pyridoxine), folic acid, vitamin B12 (cyanocobalamin), vitamin K1, vitamin K2, especially menaquinone 7-10, and biotin. Minerals include, but are not limited to, iron salts, copper salts, calcium salts such as calcium carbonate, calcium phosphate, calcium glycerophosphate; magnesium salts such as magnesium phosphate, magnesium sulphate (dihydrate) or magnesium oxide; zinc salts such as zinc citrate; selenium salts such as sodium selenate; potassium iodide; manganese salts such as manganese sulphate; molybdate salts such as sodium molybdate; chromium salts such as chromium chloride; sodium chloride and potassium chloride.

The composition according to the present invention can be used as nutritional supplement or as dietary supplement for balancing the blood lipid level, preventing or reducing the risk of the development of atherosclerotic changes, disorders or diseases in a patient. The inventive composition can also be used as nutritional or as dietary supplement for developing and maintaining the cognitive functions connected with e.g. eyes, memory, language etc. or for alleviating and/or preventing blood vessel diseases, cardiovascular, cerebrovascular and nervous diseases such as e.g. hypertension, cardiac infarction, Alzheimer, Parkinson and depression, or for alleviating hormonal, immunologic disorders or obesity. Furthermore, it can be used as nutritional or dietary supplement to support treatments of diabetes, cancer and/or inflammatory affections. A patient, for the purpose of this invention, is a mammal, including a human. The use as a nutritional or dietary supplement is especially preferred for pregnant women, children and elderly persons.

A further aspect of the invention is a composition according to the claims for use in balancing the blood lipid level, preventing or reducing the risk of the development of atherosclerotic changes, disorders or diseases, developing and maintaining the cognitive functions connected with e.g. eyes, memory, language etc., for alleviating and/or preventing blood vessel diseases, cardiovascular, cerebrovascular and nervous diseases such as e.g. hypertension, cardiac infarction, Alzheimer, Parkinson and depression, alleviating hormonal, immunologic disorders or obesity supporting treatments of diabetes, cancer and/or inflammatory affections by administering the inventive composition as nutritional supplement or as dietary supplement to a patient which is, for the purpose of this invention, a mammal, including a human, especially pregnant women, children and elderly persons.

The composition according to the invention is administered orally one or more, preferably up to three, more preferably up to two times per day. With each administration the number of dosage forms taken in at the same time should not exceed two.

Nevertheless, it may in some cases be advantageous to deviate from the amounts specified, depending on body weight, individual behaviour toward the active ingredient, type of preparation and time or interval over which the administration is effected. For instance, less than the aforementioned minimum amounts may be sufficient in some cases, while the upper limit specified has to be exceeded in other cases.

Ingredients of the oral dosage form are those which are accepted for pharmaceuticals and nutritional supplements and physiologically unobjectionable, for example: as fillers cellulose derivatives (e.g. microcrystalline cellulose), sugars (e.g. lactose), sugar alcohols (e.g. mannitol, sorbitol), inorganic fillers (e.g. calcium phosphates), binders (e.g. polyvinylpyrrolidone, gelatin, starch derivatives and cellulose derivatives), and all other excipients required to produce formulations of pharmaceuticals and nutritional supplements of the desired properties, e.g. lubricants (magnesium stearate), e.g. disintegrants (e.g. crosslinked polyvinylpyrrolidone, sodium carboxymethylcellulose), e.g. wetting agents (e.g. sodium lauryl sulphate), e.g. release-slowing agents (e.g. cellulose derivatives, polyacrylic acid derivatives), e.g. coloured pigments, e.g. effervescent couples.

Excipients for pharmaceuticals and nutritional supplements familiar to the skilled person are also described for example in the following handbook: "Handbook of Pharmaceutical Excipients", Rowe R.C., Sheskey P.J. & Weller, P.J., American Pharmaceutical Association, Washington, 4th edition 2003.

The composition according to the invention includes a tablet, a coated tablet, a multilayer tablet, a tablet with coated granules, a core tablet, a coat-core tablet or immediate, slow and timed release preparations.

Preference is given to a tablet, a coated tablet or a multilayer tablet. Most preferably the composition is a tablet wherein the activated charcoal is dispersed in the matrix of the tablet, a three-layer tablet wherein the activated charcoal is in the two outer layers, or a coated tablet wherein the activated charcoal is in the coating.

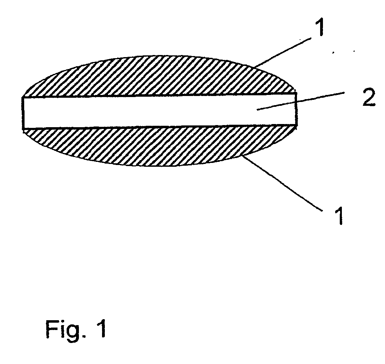

Fig. 1 shows a three-layer tablet wherein the activated charcoal is in the two outer layers (1) and the PUFA is in the inner layer (2).

The composition mentioned herein is produced by general standard processes. E.g. tablets can be produced by mixing and/or granulating the active ingredients together with the excipients to form a blend which is finally pressed to tablets. Optionally different blends containing different ingredients and excipients can be premixed and combined to a final blend which is then pressed to tablets, or the premixtures can be pressed to a multilayer tablet. In the case of an effervescent formulation the acid/base couple can be added e.g. to final blend or the acid and the base are added at different times to the blend. Also the base and the acid can be added to different blends which are finally combined.

Advantage of the composition of the present invention is that there is not any bad or fishy smell, odor or taste, even after storage of several weeks, and the inventive composition can do without any flavour or antioxidant.

The composition according to the present invention shows a good and/or fast resorption of the PUFA after administration of the composition. Furthermore, the composition facilitates a quick intake, optionally without water or drink.

The composition according to the invention shows an acceptable hardness and friability to be manufactured without affecting the beadlet integrity, i.e. no PUFA exudates from the tablet matrix.

Advantage of the composition of the present invention is that for the preparation of the composition a complex and expensive taste masking technology known in the prior art such as coating of granules, adding of antioxidants, or putting the PUFA into a capsule is not needed. The composition of the present invention can be prepared by simple and well-known standard procedures. Another well-known taste masking method is the addition of flavours in order to cover and mask the bad smell. This taste masking method is normally restricted to only a few applicable flavours which have to be selected in each case. However, flavouring ingredients are not needed for taste masking in the composition of the present invention.

### Examples:

### Example 1:

A three-layered tablet according to Fig. 1 consisting of the two outer layers A and the inner layer B wherein the inner layer B consists of:
• 600 mg of dry fish oil powder (omega-3, 18:12) including 28.2 mg of EPA and 20 mg of DHA
• 64 mg of anhydrous dibasic calcium phosphate
• 40 mg of cyclodextrin
• 40 mg of hydroxypropylcellulose
• 40 mg of bentonite
• 4 mg of silicon dioxide
• 4 mg of magnesium stearate
• 8 mg of desodorized rosemary extract
and the two outer layer A consists each of:
• 25 mg of activated charcoal (90% of the particles have a particle size less then 100 µm, loss on ignition at 600°C: ≤1%, loss on drying: ≤ 10%)
• 25 mg of hydroxypropylcellulose
• 50 mg of microcrystalline cellulose

### manufacturing process of example 1:

The ingredients of layer B are mixed together in a tumble mixer for 20 min and 5 min. before the end the lubricant magnesium stearate is added to give the final blend 1 which is than filled into the feeder 1 of the device for multilayer tablet. The ingredients of layer A are mixed together in a tumble mixer for 20 min to give the final blend 2 which is than filled into the feeder 2 and 3 of the device for multilayer tablet. The threelayer tablet is than pressed by a rotary press.

### Example 2:

A tablet consisting of:
• 600 mg of dry fish oil powder (omega-3, 18:12) including 28.2 mg of EPA and 20 mg of DHA
• 64 mg of anhydrous dibasic calcium phosphate
• 40 mg of cyclodextrin
• 40 mg of hydroxypropylcellulose
• 40 mg of bentonite
• 4 mg of silicon dioxide
• 4 mg of magnesium stearate
• 8 mg of desodorized rosemary extract
• 50 mg of activated charcoal (90% of the particles have a particle size less then 100 µm, loss on ignition at 600°C: ≤ 1%, loss on drying: ≤ 10%)
• 50 mg of hydroxypropylcellulose
• 100 mg of microcrystalline cellulose

### manufacturing process of example 2:

### Direct compression process :

All ingredients are mixed together in a tumble mixer for 20 min. and optionally 5 min. before the end the lubricant magnesium stearate is added. The final blend is pressed into tablets with a rotary press.

### Example3:

A coated tablet consisting of a core consisting of:
• 600 mg of dry fish oil powder (omega-3, 18:12) including 28.2 mg of EPA and 20 mg of DHA
• 348 mg of anhydrous dibasic calcium phosphate
• 48 mg of cyclodextrin
• 60 mg of hydroxypropylcellulose
• 60 mg of bentonite
• 60 mg of povidone (kollidon VA 60 fine)
• 6 mg of silicon dioxide
• 6 mg of magnesium stearate
• 12 mg of desodorized rosemary extract
and a coating consisting of:
• 15.5 mg of activated charcoal(90% of the particles have a particle size less then 100 µm, loss on ignition at 600°C: ≤1%, loss on drying: ≤ 10%)
• 15 mg of schellac
• 22.5 mg of hydroxypropylmethylcellulose
• 9 mg of triacetine

### manufacturing process of example 3:

All ingredients of the core are mixed together in a tumble mixer for 20 min. and optionally 5 min. before the end the lubricant magnesium stearate is added. The blend is pressed into tablets with a rotary press. Thereafter the tablets are coated with a coating suspension consisting of activated charcoal, shellac, hydroxypropylmethylcellulose, triacetine and purified water

### Results:

Examples 1, 2 and 3 do not show any bad or fishy taste or smell.

## Claims

1. Oral composition comprising fish oil, perilla oil or polyunsaturated fatty acids (PUFA) and activated charcoal.

2. Composition of claim 1 without fishy or bad smell, odour or taste.

3. Composition of claim 1 or 2, wherein the PUFA are comprised in fish oil or perilla oil, or are omega-3 fatty acids, omega-6-fatty acids, arachidonic acid, linoleic acid, alpha-linoleic acid, dihomogammalinoleic acid, eicosapentenoic acid (EPA), docosahexenoic acid (DHA) or mixtures thereof.

4. Composition of claim 1 or 3, wherein the fish oil contains eicosapentenoic acid (EPA) and docosahexenoic acid (DHA).

5. Composition of claim 1 or 4, wherein the fish oil is fish oil powder or granulate.

6. Composition of any of claims 1 to 5, wherein the amount of the fish oil, perilla oil or PUFA is of 5 % to 70% by weight of the composition.

7. Composition of any of claims 1 to 6, wherein the amount of the fish oil, perilla oil or PUFA in the composition is 100 mg - 1000 mg.

8. Composition of any of claims 1 to 7 comprising at least one further mineral and/or vitamin.

9. Composition of any of claims 1 to 8 which is an oral nutritional or dietary supplement.

10. Composition of any of claims 1 to 9 which is a tablet, a coated tablet or a multilayer tablet.

11. Composition of any of claims 1 to 10 which is a tablet wherein the activated charcoal is dispersed in the matrix of the tablet.

12. Composition of any of claims 1 to 11 which is a three-layer tablet wherein the activated charcoal is in the two outer layers.

13. Composition of any of claims 1 to 12 which is a coated tablet wherein the activated charcoal is in the coating.

14. Process for manufacturing of the composition according to any of claims 1 to 13.

15. Composition according to any of claims 1 to 13 as nutritional supplement or as dietary supplement for use in preventing or reducing the risk of the development of atherosclerotic changes, disorders or diseases, for developing and maintaining the cognitive functions, for alleviating and/or preventing blood vessel diseases, cardiovascular, cerebrovascular and nervous diseases, for alleviating hormonal, immunologic disorders or obesity, for supporting treatments of diabetes, cancer and/or inflammatory affections.

16. Composition according to any of claims 1 to 13 for use in preventing or reducing the risk of the development of atherosclerotic changes, disorders or diseases, developing and maintaining the cognitive functions, alleviating and/or preventing blood vessel diseases, cardiovascular, cerebrovascular and nervous diseases, alleviating hormonal, immunologic disorders or obesity, supporting treatments of diabetes, cancer and/or inflammatory affections.

## Patentansprüche

1. Orale Zusammensetzung, enthaltend Fischöl, Perillaöl oder mehrfach ungesättigte Fettsäuren (Polyunsaturated Fatty Acids, PUFA) und Aktivkohle.

2. Zusammensetzung nach Anspruch 1 ohne fischartigen oder schlechten Geruch, Duft oder Geschmack.

3. Zusammensetzung nach Anspruch 1 oder 2, wobei es sich bei den PUFA um solche enthalten in Fischöl oder Perillaöl, oder um Omega-3-Fettsäuren, Omega-6-Fettsäuren, Arachidonsäure, Linolsäure, Alpha-Linolsäure, Dihomogamma-Linolsäure, Eicosapentensäure (EPA), Docosahexensäure (DHA) oder Mischungen davon handelt.

4. Zusammensetzung nach Anspruch 1 oder 3, wobei es sich bei dem Fischöl um eines, das Eicosapentensäure (EPA) und Docosahexensäure (DHA) enthält, handelt.

5. Zusammensetzung nach Anspruch 1 oder 4, wobei es sich bei dem Fischöl um Fischölpulver oder -granulat handelt.

6. Zusammensetzung nach einem der Ansprüche 1 bis 5, wobei die Menge an Fischöl, Perilaöl oder PUFA 5 Gew.-% bis 70 Gew.-% der Zusammensetzung beträgt.

7. Zusammensetzung nach einem der Ansprüche 1 bis 6, wobei die Menge an Fischöl, Perillaöl oder PUFA in der Zusammensetzung 100 -1000 mg beträgt.

8. Zusammensetzung nach einem der Ansprüche 1 bis 7, enthaltend wenigstens einen weiteren Mineralstoff und/oder ein weiteres Vitamin.

9. Zusammensetzung nach einem der Ansprüche 1 bis 8, bei der es sich um ein orales Nahrungs- bzw. Ernährungsergänzungsmittel handelt.

10. Zusammensetzung nach einem der Ansprüche 1 bis 9, bei der es sich um eine Tablette, ein Dragee oder eine mehrschichtige Tablette handelt.

11. Zusammensetzung nach einem der Ansprüche 1 bis 10, bei der es sich um eine Tablette handelt, in der die Aktivkohle in der Matrix der Tablette dispergiert vorliegt.

12. Zusammensetzung nach einem der Ansprüche 1 bis 10, bei der es sich um eine dreischichtige Tablette handelt, wobei sich die Aktivkohle in den beiden äußeren Schichten befindet.

13. Zusammensetzung nach einem der Ansprüche 1 bis 12, bei der es sich um ein Dragee handelt, wobei sich die Aktivkohle im Überzug befindet.

14. Verfahren zur Herstellung der Zusammensetzung nach einem der Ansprüche 1 bis 13.

15. Zusammensetzung nach einem der Ansprüche 1 bis 13 als Nahrungsergänzungsmittel oder als Ernährungsergänzungsmittel zur Verwendung bei der Prävention oder der Verminderung des Risikos des Entstehens von arteriosklerotischen Veränderungen, Erkrankungen oder Krankheiten, zur Entwicklung und Aufrechterhaltung der kognitiven Funktionen, zur Linderung und/oder Prävention von Blutgefäßerkrankungen, Herz-Kreislauf-Krankheiten, zerebrovaskulären Krankheiten und Nervenkrankheiten, zur Linderung von hormonellen immunologischen Störungen oder Obesitas, für unterstützende Behandlungen von Diabetes, Krebs und/oder entzündlichen Leiden.

16. Zusammensetzung nach einem der Ansprüche 1 bis 13 zur Verwendung bei der Prävention oder der Verminderung des Risikos des Entstehens von arteriosklerotischen Veränderungen, Erkrankungen oder Krankheiten, zur Entwicklung und Aufrechterhaltung der kognitiven Funktionen, zur Linderung und/oder Prävention von Blutgefäßerkrankungen, Herz-Kreislauf-Krankheiten, zerebrovaskulären Krankheiten und Nervenkrankheiten, zur Linderung von hormonellen immunologischen Störungen oder Obesitas, für unterstützende Behandlungen von Diabetes, Krebs und/oder entzündlichen Leiden.

## Revendications

1. Composition orale comprenant de l'huile de poisson, de l'huile de périlla ou des acides gras polyinsaturés (PUFA) et du charbon actif.

2. Composition de la revendication 1 sans arôme, odeur ou goût de poisson ou désagréable.

3. Composition de la revendication 1 ou 2, dans laquelle les PUFA sont compris dans l'huile de poisson ou l'huile de périlla, ou sont des acides gras oméga-3, des acides gras oméga-6, l'acide arachidonique, l'acide linoléique, l'acide alpha-linoléique, l'acide dihomogammalinoléique, l'acide eicosapenténoïque (EPA), l'acide docosahexénoïque (DHA) ou des mélanges de ceux-ci.

4. Composition de la revendication 1 ou 3, dans laquelle l'huile de poisson contient de l'acide eicosapenténoïque (EPA) et de l'acide docosahexénoïque (DHA).

5. Composition de la revendication 1 ou 4, dans laquelle l'huile de poisson est de la poudre ou du granulat d'huile de poisson.

6. Composition de l'une quelconque des revendications 1 à 5, dans laquelle la quantité de l'huile de poisson, de l'huile de périlla ou des PUFA est de 5 % à 70 % en poids de la composition.

7. Composition de l'une quelconque des revendications 1 à 6, dans laquelle la quantité de l'huile de poisson, de l'huile de périlla ou des PUFA dans la composition est de 100 mg à 1000 mg.

8. Composition de l'une quelconque des revendications 1 à 7 comprenant au moins un autre minéral et/ou une vitamine.

9. Composition de l'une quelconque des revendications 1 à 8 qui est un supplément nutritionnel ou alimentaire oral.

10. Composition de l'une quelconque des revendications 1 à 9 qui est un comprimé, un comprimé enrobé ou un comprimé multicouche.

11. Composition de l'une quelconque des revendications 1 à 10 qui est un comprimé dans lequel le charbon actif est dispersé dans la matrice du comprimé.

12. Composition de l'une quelconque des revendications 1 à 11 qui est un comprimé à trois couches dans lequel le charbon actif est dans les deux couches extérieures.

13. Composition de l'une quelconque des revendications 1 à 12 qui est un comprimé enrobé dans lequel le charbon actif est dans l'enrobage.

14. Procédé de fabrication de la composition selon l'une quelconque des revendications 1 à 13.

15. Composition selon l'une quelconque des revendications 1 à 13 en tant que supplément nutritionnel ou en tant que supplément alimentaire pour utilisation dans la prévention ou la réduction du risque du développement de changements, troubles ou maladies athérosclérotiques, le développement et le maintien des fonctions cognitives, l'atténuation et/ou la prévention des maladies vasculaires, des maladies cardiovasculaires, cérébrovasculaires et nerveuses, l'atténuation des troubles hormonaux, immunologiques ou l'obésité, en appui de traitements du diabète, du cancer et/ou d'affections inflammatoires.

16. Composition selon l'une quelconque des revendications 1 à 13 pour utilisation dans la prévention ou la réduction du risque du développement de changements, troubles ou maladies athérosclérotiques, le développement et le maintien des fonctions cognitives, l'atténuation et/ou la prévention des maladies vasculaires, des maladies cardiovasculaires, cérébrovasculaires et nerveuses, l'atténuation des troubles hormonaux, immunologiques ou l'obésité, en appui de traitements du diabète, du cancer et/ou d'affections inflammatoires.
